# FASCICULE DE BREVET EUROPEEN

(11) **EP 2 619 165 B1**
(45) Date de publication et mention de la délivrance du brevet: **06.06.2018**
(21) Numéro de dépôt: 11771240.6
(22) Date de dépôt: 21.09.2011
(51) Int. Cl.: C07C 51/41

(54) **PROCÉDÉ DE PRÉPARATION D'UN COMPLEXE D'ACIDE ET D'UN MÉTAL**
VERFAHREN ZUR HERSTELLUNG EINES KOMPLEXES AUS EINER SÄURE UND EINEM METALL
METHOD FOR PREPARING A COMPLEX OF AN ACID AND A METAL

(30) Priorité: 22.09.2010 FR 1057605
(43) Date de publication de la demande: 31.07.2013
(73) Titulaire: Adisseo Ireland Limited, Dublin 2 (IE)
(72) Inventeur: LE THIESSE, Jean-Claude, 42100 Saint-Etienne (FR); REY, Patrick, 69003 Lyon (FR); HENRYON, Vivien, 69007 Lyon (FR)
(74) Mandataire: Delorme, Nicolas
(86) Numéro de dépôt international: PCT/FR2011/052171
(87) Numéro de publication internationale: WO 2012/038660

(56) Documents cités:
- EP-A1- 0 049 057
- EP-A1- 1 260 496
- DE-A1- 19 707 380
- US-A1- 2007 227 399

## Description

L'invention concerne un procédé de préparation d'un complexe d'un acide et d'au moins un métal. Plus précisément, ce procédé vise la fabrication d'un complexe métallique d'un acide substitué en position alpha du groupement carboxylique, par un groupement aminé ou hydroxyle. Le procédé de l'invention trouve un intérêt tout particulier dans l'obtention d'un complexe métallique d'un acide choisi parmi la méthionine, l'acide 2-hydroxy-4-méthylthiobutanoïque (HMTBA) et l'acide lactique.

Le carbone porteur du groupement aminé ou hydroxyle et du groupement carboxylique étant asymétrique, on entend par acide, chacun de ses isomères, L- ou D-, ou leurs mélanges et notamment le racémique.

La méthionine, acide aminé essentiel, et l'HMTBA, un analogue de la méthionine, trouvent des applications largement étendues chez l'homme en tant que complément alimentaire ou médicament, ainsi qu'en nutrition animale. Leurs sels métalliques, par exemple calciques ou de zinc, sous forme solide, peuvent être préférés. Ceux-ci permettent aussi de combler des carences en éléments ou oligoéléments. Le sel d'HMTBA le plus connu est le sel dicalcique, comprenant deux moles d'équivalent d'HMTBA par mole de calcium répondant à la formule (HMTBA)₂ Ca.

On connait selon EP140865A, un procédé de préparation de sels de calcium de HMTBA, consistant en plus de deux et moins de dix moles d'équivalent d'HMTBA par mole de calcium. Ces sels sont obtenus en mettant en réaction le HMTBA avec une source de calcium choisie parmi l'oxyde calcium (CaO), l'hydroxyde de calcium (Ca(OH)₂), le carbonate de calcium (CaCO₃) ainsi qu'un sel de HMTBA, par exemple le sel (HMTBA)₂ Ca. L'HMTBA est généralement en solution aqueuse fortement concentrée, à laquelle la source de calcium est mélangée, puis le milieu réactionnel ainsi obtenu est séché à une température de l'ordre de 70°C. Le milieu réactionnel du HMTBA avec la source de calcium est cependant très visqueux et collant : il est donc très difficile à homogénéiser dans des mélangeurs ou réacteurs équipés de systèmes d'agitation classiques et, en fin de réaction, il est nécessaire de procéder à un séchage in-situ pour pouvoir vidanger le réacteur. Un recyclage de sel de calcium de HMTBA, par exemple le sel (HMTBA)₂ Ca, à la source de calcium avant de la mettre en contact avec le HMTBA permet d'améliorer la consistance du milieu réactionnel et facilite la mise en oeuvre du procédé. Mais, comme enseigné par US4335257, cette amélioration est observée pour une proportion pondérale d'au moins 20% dudit sel par rapport au milieu réactionnel, et pour parvenir à une consistance acceptable il peut être nécessaire que cette proportion atteigne 80% du milieu réactionnel. Un tel taux de recyclage de produit fini dans le mélange réactionnel réduit considérablement la productivité d'une installation industrielle et contraint à un fort surdimensionnement du mélangeur / réacteur pour une capacité de production souhaitée.

WO03/011822A2 propose un procédé de préparation de sels d'acide organique, notamment de calcium, à partir d'un dit acide organique et d'hydroxyde de calcium et/ou d'oxyde de calcium, dans lequel on dépose l'acide organique sur un support inerte avant de lui ajouter la source de calcium. Malgré la présence de ce support, il est indispensable d'introduire les deux réactifs par ajouts successifs pour permettre un séchage du milieu réactionnel entre deux ajouts. Ce mode opératoire rallonge notablement le temps de séjour dans le mélangeur et contraint également à un fort surdimensionnement dudit mélangeur pour une capacité de production donnée. En outre, le support inerte se retrouve dans le produit fini sec où il représente de 30 à 50% en poids de la masse totale, ce qui réduit d'autant le titre en matière active et génère ainsi des surcoûts à l'utilisation du produit (stockage, transport, dosage, ...).

Comme il ressort de l'art antérieur, la préparation de sels d'acide organique et notamment d'HMTBA contraint de recourir à un subterfuge, généralement l'addition d'un composé dans le milieu réactionnel, afin d'en améliorer la consistance et de rendre possible sa manipulation.

EP1260496A1 décrit un procédé industriel de préparation en continu de sels métalliques d'acide gras, dans une extrudeuse qui comprend une zone d'alimentation des réactifs, acides gras et oxyde ou hydroxyde métallique, et d'eau, une zone de réaction et une zone de refroidissement.

US2007/0227399A1 divulgue une composition pour la conservation du bois à base de sels métalliques de HMBA, tel qu'un sel de calcium, qui possède une activité antimicrobienne. Ces sels sont obtenus par des techniques classiques.

Les auteurs de la présente invention ont mis au point un procédé simple de préparation d'un ou de sels d'HMTBA, qui permet de contourner les obstacles précités, sans pour autant faire appel à un support ou autre excipient.

Ainsi, on apporte selon l'invention un procédé de préparation d'un complexe d'un acide choisi parmi la méthionine, l'acide 2-hydroxy-4-méthylthiobutanoïque (HMTBA) et l'acide lactique, et d'au moins un métal, à partir dudit acide et d'une source métallique minérale, procédé selon lequel l'acide et la source métallique minérale sont mis en réaction dans une extrudeuse.

Les auteurs ont découvert que la cinétique de la réaction précitée était parfaitement compatible avec cette technologie continue malgré le temps de séjour ne dépassant pas quelques minutes, même généralement moins d'une minute. En effet, cette technologie permet de maximiser l'intensité de malaxage des réactifs et d'obtenir un mélange parfaitement homogène en dépit de la viscosité élevée. Ainsi, le rendement de la réaction est excellent et les sels formés sont quasiment dépourvus d'oligomères résiduels. De plus, le caractère auto-nettoyant des vis de l'extrudeuse permet de convoyer aisément le milieu réactionnel malgré son caractère très collant sans qu'il soit besoin de faire appel à un quelconque support inerte ni à un recyclage de produit fini à l'alimentation de l'extrudeuse.

Ce procédé n'a recours à aucun traitement préalable des réactifs, acide et source métallique, ni de leur mélange. En particulier il ne nécessite l'apport d'aucun solvant, ni de tout autre additif ou recyclage de produit fini et il ne requiert pas non plus de préchauffage des réactifs. Ce procédé permet donc de s'affranchir de toute étape de manipulation des réactifs, ainsi que de leur mélange qui constitue directement le milieu réactionnel. Seul un ajout d'eau peut être nécessaire à l'alimentation de l'extrudeuse dans le cas où les deux réactifs n'en contiennent pas suffisamment sous leur forme initiale ; c'est notamment le cas lorsque les deux réactifs sont des solides. La quantité d'eau ajoutée est calculée de façon à ce que la quantité totale d'eau engagée dans l'extrudeuse soit comprise entre 5 et 20%, préférentiellement entre 10 et 15%.

Avant d'aborder plus en détails, la description du procédé de l'invention, une définition de certains termes employés est présentée.

Par métal selon l'invention, on entend tout élément du tableau périodique, capable de former un ou des cations, et appartenant aux groupes des métaux notamment des métaux alcalins, alcalino-terreux et de transition et des métalloïdes.

Par complexe selon l'invention, on entend un composé comprenant au moins un atome de métal et au moins une molécule d'un acide tel que défini ci-dessus, dans lequel au moins un atome de la molécule d'acide est lié à l'atome de métal ou aux atomes de métal pour la préparation de sels mixtes, par une liaison chimique ou une interaction chimique. A titre d'illustration, une ou de telles liaisons ou une ou de telles interactions chimiques sont choisies parmi les liaisons ioniques, les liaisons de coordination, des liaisons de Van der Waals... Un complexe selon l'invention peut comprendre plusieurs des composés ci-dessus décrits. A titre d'illustration, un complexe de l'invention peut être représenté par la formule (Acide)ₙXₚYₚ où X et Y représentent indépendamment l'un de l'autre un métal, n varie de 1 à 10, et p et q varient de 0 à 10, la somme de p et q variant de 1 à 10. La présente invention est plus particulièrement illustrée par la préparation de sels mais le procédé de l'invention s'applique à tout complexe tel que défini ci-dessus.

L'expression « quasiment dépourvu d'oligomères » se rapportant au complexe obtenu selon l'invention, signifie que le procédé ne conduit généralement pas à une teneur en oligomères résiduelle de plus de 0,4%, voire même de plus de 0,1%. C'est ce qui est en effet observé lorsque l'acide est l'HMTBA.

Divers dispositifs ont la capacité de réaliser les conditions de malaxage du milieu réactionnel selon l'invention. A titre d'exemples, on peut citer les extrudeuses bi-vis, corotatives ou contrarotatives, les malaxeurs monovis. Ces technologies se caractérisent un temps de séjour relativement court, quelques minutes au maximum, souvent moins d'une minute. Par voie de conséquence, la quantité de produit contenu dans l'appareil est faible en regard de l'énergie mécanique dissipée et de la surface d'échanges thermiques offerte. Il en résulte un traitement mécanique du milieu réactionnel au cours duquel les transferts de masse et de chaleur sont maximisés.

Cela conduit à une optimisation de la réaction, le taux de transformation des réactifs atteignant généralement 99%, et à l'obtention d'un complexe qui peut être directement engagé dans une étape de fractionnement et/ou de séchage, sans nécessiter de manipulation complémentaire.

L'emploi d'une extrudeuse bi-vis est préféré. Le procédé est avantageusement réalisé dans une extrudeuse bi-vis corotative, c'est-à-dire dont les vis tournent dans le même sens. Les réactifs y sont alimentés et mixés pour constituer le mélange réactionnel et y produire le complexe. Il appartient bien entendu aux connaissances générales de l'homme du métier d'adapter les caractéristiques dudit dispositif. Notamment, le profil de vis sera conçu pour assurer les trois grandes fonctions recherchées : mélange des réactifs, convoyage et malaxage intensif du milieu réactionnel. On alternera avantageusement plusieurs zones de malaxage avec des zones de convoyage. Le fourreau de l'extrudeuse est de préférence équipé de différents manchons chauffants qui permettent de faire varier la température le long de l'extrudeuse. Ainsi, les conditions réactionnelles, taux de cisaillement et température de la masse, sont optimisées tout au long de l'extrudeuse selon le degré d'avancement de la réaction. Ces conditions optimales dépendent évidemment de la nature chimique des réactifs et du complexe recherché. Il ressort des compétences générales de l'homme du métier de les déterminer.

A son extrémité, l'extrudeuse peut être équipée d'une filière. Dans ce cas, le complexe est de préférence expulsé de l'extrudeuse sous forme de joncs, non collants, directement utilisables ou, si nécessaire, aisément transportables vers une étape de séchage. Cette mise en forme du complexe autorise l'utilisation de tout type de séchoir, en particulier des séchoirs convectifs dont le rendement énergétique est très supérieur au séchage conductif imposé par les procédés de l'art antérieur.

Les débits d'alimentation des deux réactifs, acide et source de métal, sont régulés en fonction de leur nature chimique respective et du type de complexe recherché. Si nécessaire, une introduction simultanée d'eau est réalisée de façon à ce que la quantité totale d'eau injectée dans l'extrudeuse permette la formation d'une pâte. Typiquement la quantité totale d'eau représente entre 10 et 15% de la masse totale. Si l'application visée le nécessite, il est également possible d'additionner de faibles quantitiés d'excipients, amidon par exemple, pouvant atteindre 15%, préférentiellement de 100 ppm à 5% de la masse totale.

Le procédé répond avantageusement aux caractéristiques supplémentaires suivantes, qui doivent être considérées seules ou en une combinaison quelconque les unes avec les autres :
- le métal est choisi parmi Li, Na, K, Mg, Ca Mn, Fe, Co, Ni, Cu, Zn, Pt.
- la source métallique minérale est choisie parmi les hydroxydes métalliques, les laits d'hydroxyde métallique, les oxydes métalliques et les carbonates métalliques correspondants, d'origine naturelle ou non ; quand elle est d'origine naturelle, elle peut être choisie parmi les coquilles, les minerais et les roches.
- Un complexe de calcium de l'HMTBA est de préférence obtenu selon l'invention ; lorsque le métal est le calcium, la source de calcium est avantageusement choisie parmi la chaux, le lait de chaux, la chaux éteinte, l'hydrogénocarbonate de calcium et le carbonate de calcium ; quand elle est d'origine naturelle, elle peut être choisie parmi la coquille d'huître, la coquille d'escargot, la dolomie ; de manière préférée, la source de calcium est le Ca(OH)₂.
- Un complexe d'un métal choisi parmi le zinc, le cuivre et le manganèse, de la méthionine ou de l'HMTBA est aussi un complexe préféré selon l'invention; la source de métal est alors avantageusement choisie parmi l'oxyde de zinc/cuivre/manganèse, l'hydroxyde de zinc/cuivre/manganèse, une solution aqueuse d'hydroxyde de zinc/cuivre/manganèse et le carbonate de zinc/cuivre/manganèse.
- Le rapport pondéral acide/source métallique est déterminé par l'homme du métier en fonction du complexe recherché.
- La température de réaction est inférieure à 150°C, de préférence elle varie de 60 à 120°C, mieux encore de 80 à 95°C.

Le complexe ainsi obtenu peut ensuite être fractionné et/ou séché et peut subir une étape supplémentaire de formage, par exemple pour atteindre une granulométrie déterminée, par exemple par sphéronisation.

Si la gestion de l'atelier de production le nécessite, le procédé de l'invention peut faire intervenir un recyclage de produit fini à l'alimentation de l'extrudeuse, par exemple de très fines particules générées dans les étapes aval du procédé. Comme indiqué précédemment, ce recyclage est possible mais non indispensable au bon fonctionnement de l'extrudeuse compte tenu du caractère auto-nettoyant des vis et il n'y est peu recouru.

Le procédé selon l'invention présente donc de nombreux avantages :
- procédé continu
- réactifs mis en oeuvre sans aucun prétraitement, notamment dilution, ou préchauffage
- pas de recyclage de produit fini ou d'ajout d'additifs indispensables d'où un flux à traiter égal au débit de production réel
- taux de transformation de l'acide supérieur à 99% et taux d'oligomères résiduels inférieurs à 0,4%.
- mise en forme en sortie d'extrudeuse autorisant un séchage convectif
- pas d'excipient dans le produit fini d'où un titre en matière active très élevé.

Outre la facilité de mise en oeuvre du procédé, l'ensemble de ces avantages dégagent un intérêt économique : minimisation de l'investissement (nombre et taille des appareils), accroissement du rendement énergétique (procédé intensif, séchage convectif).

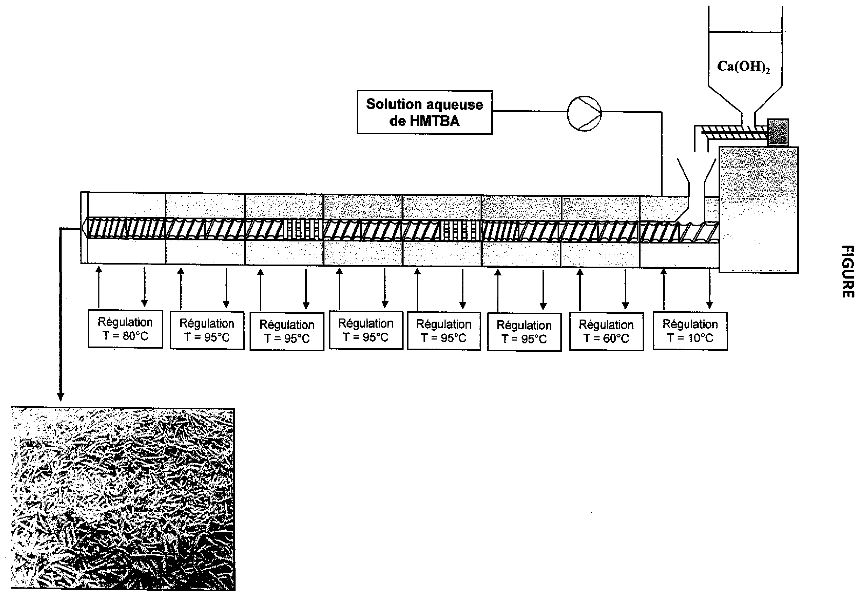

L'invention est ci-après illustrée à l'appui de l'unique figure et des exemples suivants, selon lesquels :
- la figure représente une extrudeuse dans laquelle le procédé de l'invention, en particulier celui décrit à l'exemple 1 est mis en oeuvre,
- les exemples décrivent la préparation d'un complexe calcique de HMTBA à partir d'HMTBA et de chaux éteinte, dans une extrudeuse bi-vis.

Bien entendu, le procédé de l'invention n'est pas restreint à une telle mise en oeuvre, il est notamment parfaitement adapté à la formation d'autres complexes métalliques de l'HMTBA, ainsi que tous complexes métalliques de la méthionine et de l'acide lactique.

### Exemple 1 : (référence interne pour mémoire = AGT 49)

Conformément à la figure, une extrudeuse bivis corotative CLEXTRAL BC21 équipée de 8 fourreaux, soit L/D = 32, est alimentée à :
- 1,2 kg/h de chaux éteinte GPR Rectapur commercialisée par la société VWR, par l'intermédiaire d'un doseur pondéral K-Tron,
- 5,0 kg/h d'AT88 commercialisé par la société Adisseo, par l'intermédiaire d'une pompe volumétrique.

Les deux réactifs sont à température ambiante lors de leur introduction dans l'extrudeuse. Le profil de température imposé le long de l'extrudeuse et décrit sur la figure permet de maintenir la masse réactionnelle à :
- T < 60°C dans la zone de mélange des 2 réactifs
- T = 95°C dans la zone de réaction
- T = 80°C au niveau de la filière

La vitesse de rotation des vis est de 150 tours/minute.

Le temps de séjour dans l'extrudeuse est estimé à 90 secondes.

La masse réactionnelle est extrudée à travers une filière à 1 trou de 1,6 mm de diamètre.

Les extrudats obtenus sont séchés pendant 12 heures en étuve à 60°C ; l'humidité résiduelle est de 0,5%.

L'analyse HPLC révèle un taux de dimère résiduel inférieur à 0,01%.

L'analyse DSC montre un seul signal endotherme caractéristique de la décomposition du sel double (HMTBA)₂Ca : température onset de 257°C et enthalpie de 270 J/g pour une rampe de température de 2°C/mn. L'absence de signal dans la gamme de température 120 - 140°C prouve que le produit obtenu est totalement exempt de sel (HMTBA)₄Ca.

### Exemple 2 :

Une extrudeuse bivis corotative CLEXTRAL BC21 équipée de 9 fourreaux, soit L/D = 36, est alimentée à :
- 2,4 kg/h de chaux éteinte GPR Rectapur commercialisée par la société VWR, par l'intermédiaire d'un doseur pondéral K-Tron,
- 10,0 kg/h d'AT88 commercialisé par la société Adisseo, par l'intermédiaire d'une pompe volumétrique.

Les deux réactifs sont à température ambiante lors de leur introduction dans l'extrudeuse. Le profil de température imposé le long de l'extrudeuse permet de maintenir la masse réactionnelle à :
- T < 60°C dans la zone de mélange des 2 réactifs
- T = 105°C dans la zone de réaction
- T = 105°C au niveau de la filière

La vitesse de rotation des vis est de 250 tours/minute.

Le temps de séjour dans l'extrudeuse est estimé à 50 secondes.

La masse réactionnelle est extrudée à travers une filière à 7 trous de 1,2 mm de diamètre. Les extrudats obtenus sont séchés pendant 4 heures en étuve à 60°C ; l'humidité résiduelle est de 1,5%.

L'analyse HPLC révèle un taux de dimère résiduel inférieur à 0,03%.

### Exemple 3 :

Une extrudeuse bivis corotative CLEXTRAL BC21 équipée de 9 fourreaux, soit L/D = 36, est alimentée à :
- 3,6 kg/h de chaux éteinte GPR Rectapur commercialisée par la société VWR, par l'intermédiaire d'un doseur pondéral K-Tron,
- 15,0 kg/h d'AT88 commercialisé par la société Adisseo, par l'intermédiaire d'une pompe volumétrique.

Les deux réactifs sont à température ambiante lors de leur introduction dans l'extrudeuse. Le profil de température imposé le long de l'extrudeuse permet de maintenir la masse réactionnelle à :
- T < 60°C dans la zone de mélange des 2 réactifs
- T = 105°C dans la zone de réaction
- T = 105°C au niveau de la filière

La vitesse de rotation des vis est de 450 tours/minute.

Le temps de séjour dans l'extrudeuse est estimé à 25 secondes.

La masse réactionnelle est extrudée à travers une filière à 12 trous de 0,8 mm de diamètre. Les extrudats obtenus sont séchés pendant 4 heures en étuve à 60°C ; l'humidité résiduelle est de 1,5%.

L'analyse HPLC révèle un taux de dimère résiduel inférieur à 0,06%.

### Exemple 4 :

Une extrudeuse bivis corotative CLEXTRAL Evolum HT 53 D 101 équipée de 10 fourreaux, soit L/D = 40, est alimentée à :
- 19,4 kg/h de chaux éteinte A2205 commercialisée par la société Bonargent - Goyon, par l'intermédiaire d'un doseur pondéral K-Tron,
- 76,6 kg/h d'AT88 commercialisé par la société Adisseo, par l'intermédiaire d'une pompe volumétrique.

Les deux réactifs sont à température ambiante lors de leur introduction dans l'extrudeuse. Le profil de température imposé le long de l'extrudeuse permet de maintenir la masse réactionnelle à :
- T < 60°C dans la zone de mélange des 2 réactifs
- T = 110°C dans la zone de réaction
- T = 80°C au niveau de la filière

La vitesse de rotation des vis est de 200 tours/minute.

Le temps de séjour dans l'extrudeuse est estimé à 60 secondes.

La masse réactionnelle est extrudée à travers une filière à 14 trous de 1,4 mm de diamètre. Les extrudats obtenus sont séchés pendant 2 heures en étuve à 90°C ; l'humidité résiduelle est de 2,0%.

L'analyse HPLC révèle un taux de dimère résiduel inférieur à 0,09%.

## Revendications

1. Procédé de préparation d'un complexe d'un acide choisi parmi la méthionine, l'acide 2-hydroxy-4-méthylthiobutanoïque (HMTBA) et l'acide lactique, et d'au moins un métal, à partir dudit acide et d'une source métallique minérale, **caractérisé en ce qu'**on met en réaction l'acide avec la source métallique minérale, dans une extrudeuse.

2. Procédé selon la revendication 1, **caractérisé en ce que** le métal est choisi parmi Li, Na, K, Mg, Ca, Mn, Fe, Co, Ni, Cu, Zn, Pt.

3. Procédé selon la revendication 1 ou 2, **caractérisé en ce que** la source métallique minérale est choisie parmi les hydroxydes métalliques, les laits d'hydroxyde métallique, les oxydes métalliques et les carbonates métalliques correspondants, d'origine naturelle ou non.

4. Procédé selon la revendication 3, **caractérisé en ce que** la source métallique minérale est d'origine naturelle et est choisie parmi les coquilles, les minerais et les roches.

5. Procédé selon l'une quelconque des revendications 1 à 4, **caractérisé en ce que** l'acide est l'HMTBA et le métal est le calcium, la source de calcium étant choisie parmi la chaux, le lait de chaux, la chaux éteinte, l'hydrogénocarbonate de calcium et le carbonate de calcium.

6. Procédé selon la revendication 5, **caractérisé en ce que** la source métallique minérale est choisie parmi la coquille d'huître, la coquille d'escargot, la dolomie.

7. Procédé selon l'une quelconque des revendications 3 à 6, **caractérisé en ce que** la source de calcium est le Ca(OH)₂.

8. Procédé selon l'une quelconque des revendications 1 à 4, **caractérisé en ce que** l'acide est la méthionine ou le HMTBA et un métal choisi parmi le zinc, le manganèse et le cuivre, la source de métal étant choisie parmi l'oxyde, l'hydroxyde, une solution aqueuse d'hydroxyde et le carbonate du métal correspondant.

9. Procédé selon l'une quelconque des revendications 1 à 8, **caractérisé en ce qu'**on effectue la réaction entre l'acide et la source métallique minérale dans une extrudeuse choisie parmi un malaxeur monovis et une extrudeuse bi-vis.

10. Procédé selon l'une quelconque des revendications 1 à 7 et 9, **caractérisé en ce qu'**on effectue la réaction entre l'HMTBA et Ca(OH)₂ dans une extrudeuse bi-vis.

11. Procédé selon la revendication 10, **caractérisé en ce que** l'extrudeuse bi-vis est corotative.

12. Procédé selon l'une quelconque des revendications 1 à 11, **caractérisé en ce que** la température de réaction est inférieure à 150°C.

13. Procédé selon la revendication 12, **caractérisé en ce que** la température de réaction varie de 60 à 120°C, de préférence de 80 à 95°C.

14. Procédé selon l'une quelconque des revendications 1 à 13, **caractérisé en ce qu'**il comprend une étape de fractionnement et/ou de séchage du complexe obtenu.

15. Procédé selon la revendication 14, **caractérisé en ce que** le fractionnement est réalisé par passage dans une filière ou par broyage.

16. Procédé selon l'une quelconque des revendications 1 à 15, **caractérisé en ce qu'**il comprend une étape de formage du complexe obtenu, par exemple sphéronisation.

## Patentansprüche

1. Verfahren zur Herstellung eines Komplexes einer Säure, ausgewählt aus Methionin, 2-Hydroxy-4-methylthiobuttersäure (HMTBA) und Milchsäure und mindestens einem Metall auf der Grundlage einer mineralischen Metallquelle, **dadurch gekennzeichnet, dass** die Säure mit der mineralischen Metallquelle in einem Extruder zur Reaktion gebracht wird.

2. Verfahren nach Anspruch 1, **dadurch gekennzeichnet, dass** das Metall ausgewählt ist aus Li, Na, K, Mg, Ca, Mn, Fe, Co, Ni, Cu, Zn, Pt.

3. Verfahren nach Anspruch 1 oder 2, **dadurch gekennzeichnet, dass** die mineralische Metallquelle ausgewählt ist aus den metallischen Hydroxiden, den metallische Hydroxidaufschlämmungen, den metallischen Oxiden und den entsprechenden metallischen Carbonaten natürlichen Ursprungs oder nicht.

4. Verfahren nach Anspruch 3, **dadurch gekennzeichnet, dass** die mineralische Metallquelle natürlichen Ursprungs ist und ausgewählt ist aus den Schalen, den Mineralien und den Gesteinen.

5. Verfahren nach einem beliebigen der Ansprüche 1 bis 4, **dadurch gekennzeichnet, dass** die Säure HMTBA ist und das Metall Calcium ist, wobei die Calciumquelle ausgewählt ist aus Kalk, Kalkaufschlämmung, gelöschtem Kalk, Calciumhyrogencarbonat und Calciumcarbonat.

6. Verfahren nach Anspruch 5, **dadurch gekennzeichnet, dass** die mineralische Metallquelle ausgewählt ist aus Austernschale, Schneckenschale, Dolomit.

7. Verfahren nach einem beliebigen der Ansprüche 3 bis 6, **dadurch gekennzeichnet, dass** die Kalziumquelle Ca(OH)₂ ist.

8. Verfahren nach einem beliebigen der Ansprüche 1 bis 4, **dadurch gekennzeichnet, dass** die Säure Methionin oder HMTBA und ein Metall ist, ausgewählt aus Zink, Mangan und Kupfer, wobei die Metallquelle ausgewählt ist aus Oxyd, Hydroxyd, einer wässrigen Hyroxidlösung und dem Carbonat des entsprechenden Metalls.

9. Verfahren nach einem beliebigen der Ansprüche 1 bis 8, **dadurch gekennzeichnet, dass** die Reaktion zwischen der Säure und der mineralischen Metallquelle in einem Extruder durchgeführt wird, ausgewählt aus einem Einschneckenkneter und einem Zweischneckenextruder.

10. Verfahren nach einem beliebigen der Ansprüche 1 bis 7 und 9, **dadurch gekennzeichnet, dass** die Reaktion zwischen HMTBA und Ca(OH)₂ in einem Zweischneckenextruder durchgeführt wird.

11. Verfahren nach Anspruch 10, **dadurch gekennzeichnet, dass** der Zweischneckenextruder korotativ ist.

12. Verfahren nach einem beliebigen der Ansprüche 1 bis 11, **dadurch gekennzeichnet, dass** die Reaktionstemperatur weniger als 150 °C ist.

13. Verfahren nach Anspruch 12, **dadurch gekennzeichnet, dass** die Reaktionstemperatur von 60 bis 120 °C, vorzugsweise von 80 bis 95 °C variiert.

14. Verfahren nach einem beliebigen der Ansprüche 1 bis 13, **dadurch gekennzeichnet, dass** es einen Schritt des Fraktionierens und/oder des Trocknens des erhaltenen Komplexes umfasst.

15. Verfahren nach Anspruch 14, **dadurch gekennzeichnet, dass** die Fraktionierung durch den Durchgang durch eine Düse oder durch Zerkleinerung durchgeführt wird.

16. Verfahren nach einem beliebigen der Ansprüche 1 bis 15, **dadurch gekennzeichnet, dass** es einen Schritt des Bildens des erhaltenen Komplexes umfasst, z. B. durch Spheronisierung.

## Claims

1. A method for preparing a complex of an acid chosen from among methionine, 2-hydroxy-4-methylthiobutanoic acid (HMTBA) and lactic acid, and of at least one metal, starting from the said acid and a mineral metal source, **characterized in that** the acid is placed in reaction with the mineral metal source in an extruder.

2. The method according to claim 1, **characterized in that** the metal is chosen from among Li, Na, K, Mg, Ca, Mn, Fe, Co, Ni, Cu, Zn, Pt.

3. The method according to claim 1 or 2, **characterized in that** the mineral metal source is chosen from among metallic hydroxides, metallic hydroxide milks, metallic oxides and corresponding metallic carbonates, whether or not of natural origin.

4. The method according to claim 3, **characterized in that** the mineral metal source is of natural origin and is chosen from among shells, ores and rocks.

5. The method according to any of claims 1 to 4, **characterized in that** the acid is HMTBA and the metal is calcium, the calcium source being chosen from among lime, milk of lime, slaked lime, calcium hydrogen carbonate and calcium carbonate.

6. The method according to claim 5 **characterized in that** the mineral metal source is chosen from among oyster shell, snail shell, dolomite.

7. The method according to any of claims 3 to 6, **characterized in that** the calcium source is Ca(OH)₂.

8. The method according to any of claims 1 to 4, **characterized in that** the acid is methionine or HMTBA and a metal is chosen from among zinc, manganese and copper, the metal source being chosen from among the oxide, hydroxide, aqueous hydroxide solution and the corresponding metal carbonate.

9. The method according to any of claims 1 to 8 **characterized in that** the reaction is conducted between the acid and the mineral metal source in an extruder chosen from among a single-screw mixer and a twin-screw extruder.

10. The method according to any of claims 1 to 7 and 9 **characterized in that** the reaction is conducted between HMTBA and Ca(OH)₂ in a twin-screw extruder.

11. The method according to claim 10, **characterized in that** the twin-screw extruder is co-rotating.

12. The method according to any of claims 1 to 11, **characterized in that** the reaction temperature is lower than 150°C.

13. The method according to claim 12, **characterized in that** the reaction temperature varies between 60 and 120°C, preferably between 80 and 95°C.

14. The method according to any of claims 1 to 13, **characterized in that** it comprises a fractionating and/or drying step of the complex obtained.

15. The method according to claim 14, **characterized in that** fractionation is conducted by passing through a die or by grinding.

16. The method according to any of claims 1 to 15, **characterized in that** it comprises a step for forming the complex obtained, e.g. by spheronization.
